(19) Europäisches Patentamt
European Patent Office
Office européen des brevets

(11) Publication number : **0 489 581 A2**

(12) **EUROPEAN PATENT APPLICATION**

(21) Application number : **91311258.7**

(22) Date of filing : **03.12.91**

(51) Int. Cl.$^5$ : **A61K 31/465,** A61K 7/06, A61K 7/00, A61K 7/075

(30) Priority : **04.12.90 GB 9026354**

(43) Date of publication of application :
**10.06.92 Bulletin 92/24**

(84) Designated Contracting States :
**AT BE CH DE DK ES FR GB GR IT LI NL SE**

(71) Applicant : **UNILEVER PLC**
**Unilever House Blackfriars**
**London EC4P 4BQ (GB)**
(84) **GB**

(71) Applicant : **Unilever N.V.**
**Burgemeester s'Jacobplein 1**
**NL-3015 CA Rotterdam (NL)**
(84) **BE CH DE DK ES FR GR IT LI NL SE AT**

(72) Inventor : **Best, John**
**Windmill Court**
**Sharnbrook, Bedfordshire MK44 1PG (GB)**
Inventor : **Gibson, Walter Thomas**
**8 Braid Court**
**Wellingborough, Northants NN8 6PF (GB)**
Inventor : **Wiechers, Johann Wilhelm**
**8 Lee Farm Cottages, Colworth Estate**
**Sharnbrook, Bedfordshire MK44 1LH (GB)**

(74) Representative : **Tonge, Robert James et al**
**UNILEVER PLC Patents Division P.O. Box 68**
**Unilever House**
**London EC4P 4BQ (GB)**

(54) **Cosmetic composition.**

(57) Compositions containing an ester of nicotinic acid and a cosmetically acceptable surfactant are provided. These compositions, when applied topically to the human body produce a response such as a sensory or therapeutic response, for example a tingling or warming sensation or an increase in blood flow, which persists after the composition is rinsed from the body. The compositions are particularly suitable for application to the hair and/or scalp where they may encourage hair growth. Compositions for application to the hair and/or scalp preferably additionally comprise hair benefit agents such as hair growth promoters.

EP 0 489 581 A2

## FIELD OF THE INVENTION

The invention relates to a method of treating the surface of the human body to induce a perceived sensation or benefit in the region treated; the invention further relates to a composition for topical application for producing the sensation or benefit. In particular, the invention relates to a method for treating the hair or the scalp of a human subject in order to induce a perceived sensation, particularly to the scalp, or a therapeutic benefit to the hair and/or the scalp. The invention in particular relates to a method of treating the hair and/or the scalp with an ester of nicotinic acid, optionally together with a hair and/or scalp benefit substance, and subsequently rinsing the head with water.

The invention also relates to a composition for topical application to the hair and/or the scalp which is subsequently rinsed from the head.

However, the invention is not limited to treatment of the hair and/or scalp but emcompasses compositions for topical application to other parts of the body and which are then rinsed off.

## BACKGROUND TO THE INVENTION AND PRIOR ART

Products which are intended for topical application to the hair and/or the scalp of the human subject fall into two categories:

(i) those which are applied to the hair and/or scalp, for example, lotions, creams, gels, aerosols and pomades, which are intended to enhance the appearance and/or feel of the hair and/or the scalp, and which are left in place without subsequent washing or rinsing with water; and

(ii) those which are applied to the hair and/or scalp, for example, as liquids or gels, which are intended to cleanse the hair and/or the scalp, or condition the hair and which are subsequently rinsed from the head with water.

Among those products which fall into category (i) i.e. leave-on products, are, for example, those that are intended to induce or stimulate hair growth or retard hair loss, and these products can contain, in addition to a hair growth stimulant, an ester of nicotinic acid which is believed to have a vasodilatory effect when applied to the skin, and which is also believed to enhance the activity of any hair growth promoters, such as minoxidil, that may also be present in the product.

Thus, GB 2 194 734 (L'Oreal) concerns compositions for inducing and stimulating hair growth or retarding hair loss, which comprise a pyrimidine derivative and a nicotinicester. A preferred composition comprises 6-amino-1,2-dihydro-1-hydroxy-2-amino-4-piperidinopyrimidine which influences hair regrowth, and methyl nicotinate, a vasodilator which L'Oreal state has no action in respect of hair regrowth.

Also, GB 2 176 104 (L'Oreal) concerns cosmetic compositions for treatment of hair and the scalp, particularly to promote fresh hair growth, which comprise a water-soluble poly-beta-alanine and a nicotinic ester, preferably a $C_1$-$C_6$ alkyl ester in particular the methyl ester or the benzyl ester. The compositions are presented especially in the form of non-rinse products such as lotions, hair-styling foams, hair-shaping lotions, hair-setting lotions, lotions for blow-drying or gels.

EP-A-0 243 248 (Laboratoires Pharmaceutiques Roche-Posay) discloses compositions in the form of lotions, gels, emulsions or pommades for treating baldness and hair loss which may contain 0.1 to 5% benzyl nicotinate.

EP-A-0 100 915 (Eisai Co., Ltd) discloses hair growth compositions which may contain a skin peripheral vasodilator drug, of which benzyl nicotinate and vitamin E nicotinate are mentioned as examples.

GB-A-2 216 003 (Toyama Chemical Co Ltd) describes hair restorer compositions which may contain phytyl nicotinate.

Among those products which fall into category (ii) i.e. rinse-off products, are, for example, those which are intended to cleanse the hair while at the same time treat baldness, or to condition the hair while improving its bulk.

Thus, FR 2 620 330 (Pierre Fabre Cosmetique) concerns a topical composition for use in the treatment of balding which comprises 0.1 to 5% by weight Minoxidil, 5 to 50% by weight of an alkyl carboxamide, such as N-hydroxyethyl acetamide and 0.1 to 5% by weight of a vasodilator pyridine derivative, of which methyl nicotinate is an example. The composition can also be incorporated into a shampoo. This reference, however, does not elaborate on the suggestion that it could be incorporated into a shampoo by suggesting the alleged treatment of baldness is effective, when clearly the shampoo is intended to include a rinse-off stage.

Also, EP 394 920 (Revlon) describes a hair enrichment composition and method of using it. The composition contains a hair sorbable bulking agent such as a polymer, a blood vessel dilator such as methyl or ethyl nicotinate and a scalp stimulant such as ethanol. In use, the composition is applied to the hair and subsequently rinsed with water and dried. This reference does not, however, suggest that the nicotinate can give rise to a sensory response in the scalp, even after rinsing with water, nor does it suggest that such a response can be

used to signal the promotion of a therapeutic response such as hair growth.
US-4 690 818 (Charles of the Ritz Group Ltd) discloses hair and body shampoos for hair and skin conditioning and moisturising which contain a combination of cocodimonium hydrolysed keratin and a mixture of monosaccharides and disaccharides in natural combination with amino acids. A suggested source of the mono- and disaccharides is a commercially available material which, coincidentally, contains hexyl nicotinate.
GB-A-2 049 419 (Ferrokemia Ipari Szövetkezet) discloses the use of certain nicotinic acid derivatives as ingredients of a variety of formulations including shampoos. The only specific example of a shampoo includes the nicotinic acid salt of glucosamine.

We have now discovered that certain esters of nicotinic acid can be applied to the surface of the body, in particular to the hair and/or the scalp in order to impart to the area treated a pleasing sensory response which signals to the user that a physiological or therapeutic benefit or response has been or is being achieved, even after the surface is thoroughly rinsed with water. It is particularly surprising that such a response persists even following rinsing the body surface or hair following treatment with a shampoo containing an ester of nicotinic acid, which procedure would be expected to rid the body surface or hair of the ester of nicotinic acid, along with other materials with which the surface has come into contact.

## DEFINITION OF THE INVENTION : METHOD

According to a first aspect the invention provides a method of treating the body surface of a human subject, which comprises the steps of:

i) applying to the body surface a composition comprising:

(a) an ester of nicotinic acid having the structure (1):

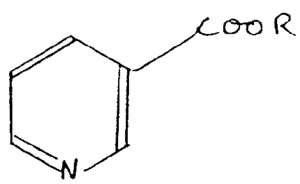

(1)

where R represents a group chosen from
branched or unbranched alkyl or alkenyl groups having from 1 to 10 carbon atoms, preferably 2 to 10 carbon atoms.
haloalkyl groups having from 1 to 6 carbon atoms,
alkoxyalkyl groups having from 2 to 6 carbon atoms,
hydroxyalkyl groups having from 1 to 6 carbon atoms,
carbamoylalkyl groups having from 2 to 7 carbon atoms,
alkylaminoalkyl groups having from 2 to 8 carbon atoms,
aminoalkyl groups having from 1 to 6 carbon atoms,
cyclohexyl,
alkylcyclohexyl groups, each alkyl group having from 1 to 4 carbon atoms,
aryl groups,
hydroxyaryl groups,
alcoxyhydroxyaryl groups
haloaryl groups,
nitroaryl groups,
tocopheryl groups,
inositol; and

(b) at least 1% by weight of a cosmetically acceptable surfactant and

ii) subsequently rinsing the body surface with water to produce a sensory response to the surface which persists after rinsing.

Preferably, the hair and/or scalp are treated with the composition which then preferably also includes (c) a hair benefit agent chosen from hair growth promoters, hair conditioners, anti-grease agents for hair, scalp hygiene agents, sunscreen agents, styling aids and bodying agents and mixtures thereof.

DISCLOSURE OF THE INVENTION : (METHOD)

According to some embodiments of the present invention a method is provided for treating any part of the human body surface in order to obtain a cosmetic or therapeutic benefit. For example, the composition may be applied to the whole, or substantial parts of the body, during bathing or showering in order that the user experiences a pleasant, warming, sensation after washing. Such a sensation may be of utility in a number of cosmetic treatments for example in the treatment of cellulite.

In preferred embodiments, however, the invention concerns a method for treating the hair and/or the scalp of the head of the human subject with a hair benefit substance, and an ester of nicotinic acid which either before or after rinsing the head with water will impart to the scalp a pleasing sensory response which signals to the user that a physiological therapeutic or cosmetic benefit or response has been or is being achieved. It is particularly surprising to note that this sensory response can persist long after rinsing the head with water, for example following a shampoo treatment, or if not immediately apparent it can become noticeable and persistent after rinsing. In either case, the persisting sensory response is indicative that the physiological or therapeutic benefit or response also continues to be effective.

The ester of nicotinic acid

The composition which is employed in the method according to the invention comprises an ester of nicotinic acid having the structure (1);

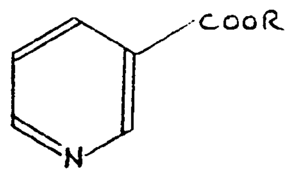

(1)

where R represents one of several generic groups, which are listed below, together with examples of preferred species illustrating each generic group.

Accordingly, examples of groups represented by R include:

i) branched or unbranched alkyl or alkenyl groups having from 2 to 10 carbon atoms, preferred examples of which are:

methyl nicotinate
ethyl nicotinate
n-propyl nicotinate
iso-propyl nicotinate
n-butyl nicotinate
iso-butyl nicotinate
tert-butyl nicotinate
n-pentyl nicotinate
n-hexyl nicotinate
n-heptyl nicotinate
n-octyl nicotinate
n-nonyl nicotinate
n-decyl nicotinate

ii) haloalkyl groups having from 1 to 6 carbon atoms, preferred examples of which are:

2-chloroethyl nicotinate
2-chloro-n-octyl nicotinate

iii) alkoxyalkyl groups having from 2 to 6 carbon atoms, preferred examples of which are:

2-methoxyethyl nicotinate
2-butoxyethyl nicotinate

iv) hydroxyalkyl groups having from 1 to 6 carbon atoms, preferred examples of which are:

2-hydroxyethyl nicotinate
3-hydroxypropyl nicotinate

v) carbamoylalkyl groups having from 2 to 7 carbon atoms, preferred examples of which are:

carbamoylmethyl nicotinate
1-carbamoylethyl nicotinate

vi) alkylaminoalkyl groups having from 2 to 8 carbon atoms, a preferred example of which is:
2-dimethylaminoethyl nicotinate
vii) aminoalkyl groups having from 1 to 6 carbon atoms, a preferred example of which is:
3-aminopropyl nicotinate
vii) cyclohexyl nicotinate
ix) alkycyclohexyl groups each alkyl group having from 1 to 4 carbon atoms, a preferred example of which is:
3,3,5-trimethylcyclohexyl nicotinate
x) Aryl groups, a preferred example of which is:
benzyl nicotinate
xi) hydroxyaryl groups, a preferred example of which is:
phenoxyethyl nicotinate
xii) alcoxyhydroxyaryl groups, a preferred example of which is:
3-methyoxythynoxyethyl nicotinate
xiii)haloaryl groups, a preferred example of which is:
p-chlorophenyl nicotinate
xiv) nitroaryl groups, a preferred example of which is:
p-nitrophenyl nicotinate
xv) tocopheryl nicotinate,
β-tocopheryl nicotinate
tocopheryl nicotinate
tocopheryl nicotinate
xvi) inositol, examples of which are:
inositol mono-nicotinate; and
inositol hexa-nicotinate. Of these, benzyl nicotinate is particularly preferred.

In order to deploy the ester of nicotinic acid in the method according to the invention, it is convenient to provide a solution or dispersion of it in a suitable cosmetically acceptable vehicle, and for this purpose, the ester of nicotinic acid and the vehicle can comprise a composition in which the ester of nicotinic acid is present in an amount of from 0.00001 to 50% by weight. Preferably, such a composition should contain from 0.001 to 20%, and most preferably from 0.001 to 10% by weight.

Hair benefit substance

The composition which is employed in the method according to preferred embodiments of the invention also comprises a hair benefit substance chosen from hair growth promoters, hair conditioners anti-grease agents for hair, scalp hygiene agents, sunscreen agents, styling aids, bodying agents, and mixtures thereof.

Hair growth promoters

Hair growth promoters include substances that are capable, following topical application to the hair and/or scalp, of maintaining, enhancing, increasing or initiating hair growth or regrowth. In particular, hair growth promoters can be useful in converting vellus hair to growth as terminal hair, or in increasing the growth rate of terminal hair or merely in maintaining terminal hair on the head where without treatment some terminal hair loss would occur.

The hair growth cycle

It should be explained that in most mammals, hair does not grow continuously, but undergoes a cycle of activity involving alternate periods of growth and rest. The hair growth cycle can be divided into three main stages, namely:

(i) the growth phase known as anagen, during which the hair follicle penetrates deep into the dermis with the cells of the bulb dividing rapidly and differentiating to form the hair,

(ii) the transitional stage known as catagen, which is heralded by the cessation of mitosis, and during which the follicle regresses upwards through the dermis and hair growth ceases,

(iii) the resting stage known as telogen, in which the regressed follicle contains a small secondary germ with an underlying ball of tightly pack dermal papilla cells.

The initiation of a new anagen phase is revealed by rapid proliferation in the germ, expansion of the dermal papilla and elaboration of basement membrane components. The hair cycle is then repeated many times until,

as a consequence of the onset of male pattern baldness, most of the hair follicles spend an increasing proportion of their time in the telogen stage, and the hairs produced become finer, shorter, and less visible; this is known as terminal to vellus transformation.

## The promotion of hair growth

Within the last decade, an increasing number of proposals has appeared in the patent literature suggesting that substances can be applied topically to the skin, particularly to the scalp, in order to maintain, promote or increase hair growth in the human subject.

Of particular note are those of Unilever, For example, the use of inhibitors of enzymes, particularly of glycosidases, whose activity is believed to curtail the hair growthcycle, are advocated in order to reverse this process and so extend the hair growth cycle, maintaining it in particular in the anagen phase.

Examples of such inhibitors include glycosaminoglycanase inhibitors such as glucarolactone as described in EP 277 428 and esters thereof as described in EP 348 184, and glucaric acid derivatives as described in EP 375 388 and glucarolactams as described in EP 334 586.

What has been lacking from all these prior proposals is a sensory signal by which the user can confirm that the hair benefit substance, such as a hair growth promoter is being delivered to the hair and/or the scalp and being retained thereon, rather than being lost, for example when the head is rinsed with water following application of a hair benefit composition.

We have, however, now discovered that the presence of an ester of nicotinic acid, as hereinbefore defined, together with a hair benefit substance, can produce a sensory signal to the scalp, for example a tingling or warming sensation, which may be associated with vasodilation of the scalp surface, to indicate that the scalp has retained the hair benefit substance even after rinsing the head with water. There is also evidence that the benefit of a hair benefit substance, such as a hair growth promoter, is enhanced with the presence of the ester of nicotinic acid, possibly due to the ability of the latter to induce vasodilation at the surface of the scalp which appears to be associated with promotion or increase in hair growth.

In a further observation, it was also discovered that ethyl nicotinate, when included in a shampoo, could itself, in the absence of any other substance known to function as a hair growth promoter, promote hair growth in its own right by stimulating blood flow.

## Examples of hair growth promoters

Particularly preferred examples of hair growth promoters include:

i) Aldonolactones, such as D-glucaro-1,4-lactone

ii) Hexosaccharic acids or salts or esters thereof, such as glucarosaccharic acid (disodium salt);

iii) Minoxidil, or a derivative thereof;

iv) $\alpha$-1,4-substituted disaccharides having the structure (2):

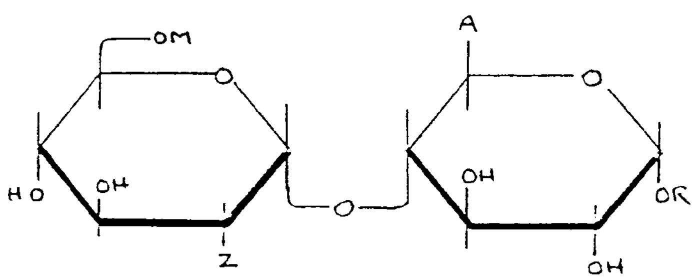

(2)

where

Z represents a functional nitrogen group, such as an azide or a group having the structure -NHB, in which B represents -H or a functional group such as acetyl or sulphate as a salt with an organic or mineral cation;

M represents -H or $SO_3M_1$, where $M_1$ is an organic or metallic cation, particularly an alkali metal; or an acetyl group;

R represents a $C_1$ or $C_4$ alkyl radical, especially methyl; or an aryl radical;

A represents a functional group such as an acid or $-COOR_1$, where $R_1$ represents -H or a $C_1$ to $C_4$ alkyl radical, especially methyl; or a metal, especially an alkali metal;

v. an oligasaccharides including at least one esterified disaccharide unit consisting of a uronic acid residue

having the structure (3):

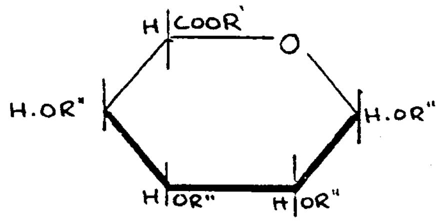

(3)

and a hexosamine residue having the structure (4):

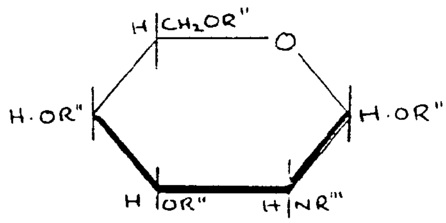

(4)

where

R′ is -H, $C_3$ to $C_{10}$ alkyl or

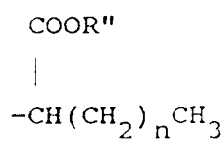

R″ is -H, $C_1$ to $C_4$ alkyl,$-CO(CH_2)_mCH_3$,$-SO_3M$,

R‴ is -H, $-CO(CK_2)_mCK_3$, or $-SO_3M$,

M is -K, or a metallic or organic cation

n is 0 or an integer of from 1 to 7, and

m is 0 or the integer 1 or 2;

the groups designated R″ being the same or different, one R″ group from each pyranose ring structure being linked by a glycosidic linkage having the configuration α-1,3, α -1,4, β-1,3 or β-1,4; and the -COOR′, -$CH_2OR2$.

and -OR" groups being of either configuration with respect to the pyranose rings;

vi) Minoxidil glucuronides,

vii) Minoxidil sulphates,

viii) Acylamino acids, such as N-acetyl glycine,

ix) Lactams, such as D-glucaro-1,5-lactam

x) Diacylglycerols, such as 1,2-dioleoyl-sn-glycerol,

xi) Acylated monosaccharides, such as 2-propionamido-2-deoxyglucose,

xii) Esters of pyroglutamic acid, such as pyroglutamic acid n-octyl ester,

xiii) Acylaldonomonolactones, such as 6-acetyl-galactono-1,4-lactone

ix) Sulphur containing amino acids, such as methionine

x) zinc salts, such as zinc gluconate

xi) pentadecanoic acid triglyceride

and mixtures thereof.

<u>Other hair benefit substances</u>

Examples of other hair benefit substances include:

Hair Conditioners

The composition which is employed in the method according to the invention can comprise a hair conditioner as a hair benefit substance.

Examples of hair conditioners include:

i. Quaternary ammonium salts having the structure (20):

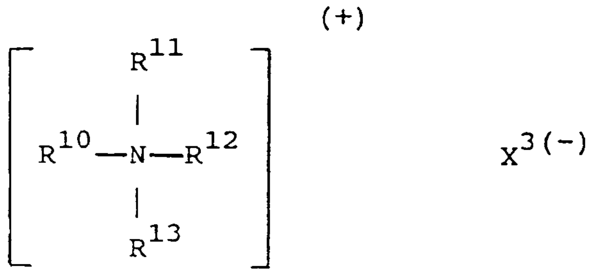

(20)

where $R^{10}$ is an alkyl group having from 8 to 30 carbon atoms.

$R^{11}$ is chosen from

a. an alkyl group having from 1 to 30 carbon atoms,

b. an aryl group having from 6 to 13 carbon atoms, and

c. $-(CH_2CH_2O)_x1\ CH_2CH_2OH$

$x^1$ is 0 or an integer of from 1 to 20;

$R^{12}$ and $R^{13}$ are each chosen from

a. an alkyl group having from 1 to 4 carbon atoms, and

b. $-(CH_2CH_2O)_x2\ CH_2\text{-}CH_2OH$ $x^2$ is 0 or an integer of from 1 to 10

$X^3$ is a counterion chosen from halogens, $-CH_3OSO_3$, $-CH_3CH_2OSO_3$ and $-H_2PO_4$.

ii. Alkyl pyridinium salts having the structure (21):

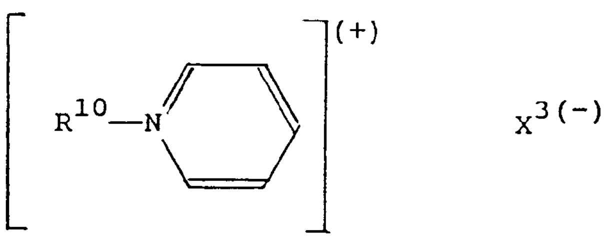

(21)

iii. N-acyl cocaminoformyl methyl pyridinium salts having the structure (22):

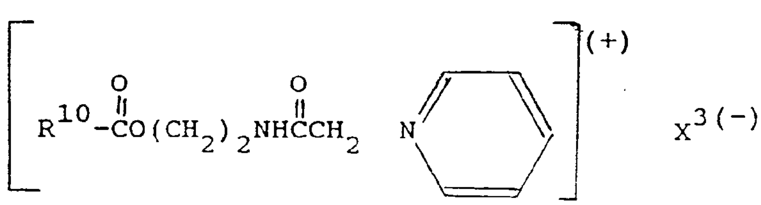

(22)

specific examples of which are:

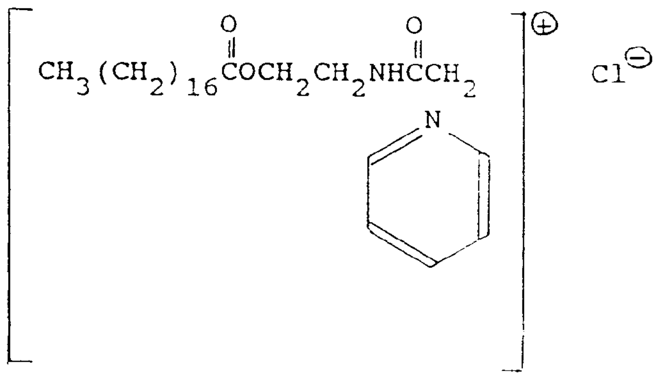

CTFA: Steapyrium chloride, available as Emcol E-607S; and

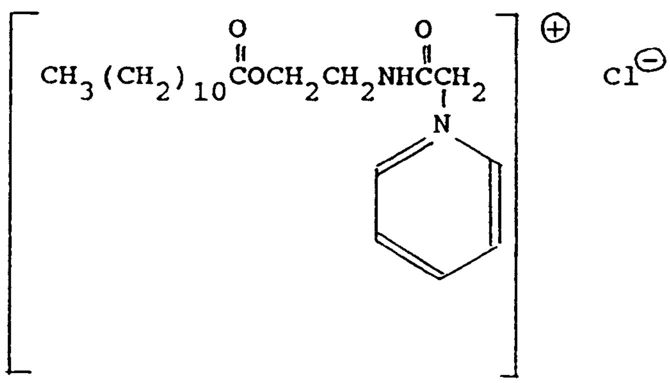

CTFA: Lapyrium chloride, available as Emcol E-607L.

iv. Acyl amido alkyl quaternary ammonium salts having the structure (23):

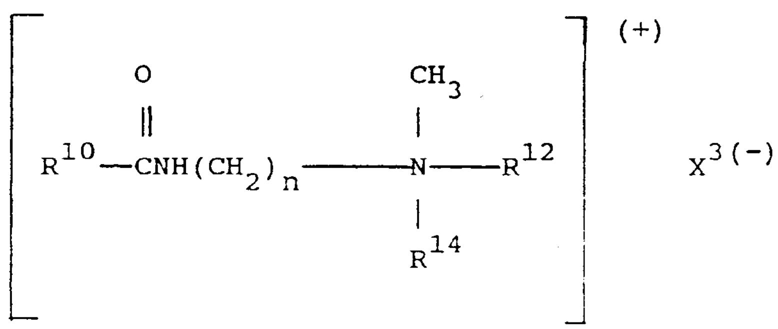

(23)

where $R^{14}$ is chosen from

a. $-(CH_2CH_2O)_x2\ CH_2CH_2OH$

b. $-CH_2CONH_2$, and

c. $-(CH_2)_x3\ NHCOR^{10}$

d. an alkyl group having from 1 to 4 carbon atoms

e. an aryl group having from 6 to 13 carbon atoms, and

f.

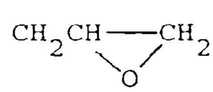

$x^3$ is 2 or 3.

Specific examples of which are (24):

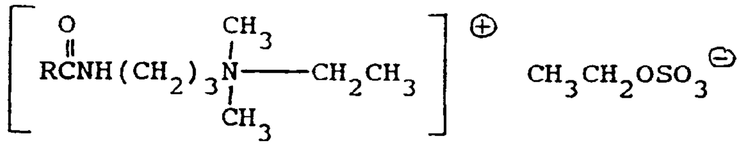

(24)

where RCO represents a soya acid radical. soyamidopropyl CTFA: ethyldimonium ethosulfate, available as Schercoquat SOAS (Sher); and (25):

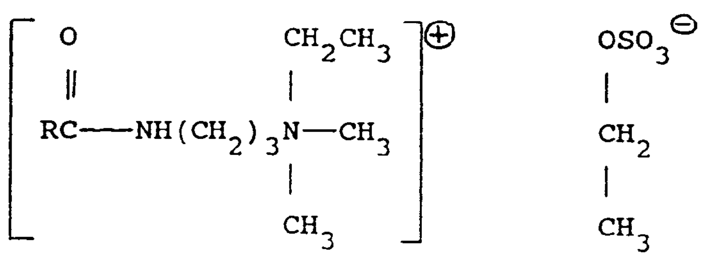

(25)

where RCO represents lanolin acid radical.
CTFA: Quaternium 33, available as Lanoquat 50.

Specific examples of preferred quaternary ammonium hair conditioning agents include:

Dimethyl di(hydrogenated tallow) ammonium chloride, also known as Quaternium -18 (CTFA)
Cetyl trimethyl ammonium chloride, also known as Cetrimonium chloride (CTFA)
Benzyl dimethyl stearyl ammonium chloride, also known as Stearalkonium chloride (CTFA)
Lauryl dimethyl benzyl ammonium chloride, also known as Lauralkonium chloride (CTFA)
Dicetyl dimethyl ammonium chloride, also known as Dicetyldimonium chloride (CTFA)

as identified in the CTFA Cosmetic Ingredient Dictionary, Third Edition, 1982, published by the Cosmetic Toiletry and Fragrance Association, Inc., hereinafter referred to as the CTFA Dictionary, and which Dictionary names other cosmetic ingredients named hereinafter.

Further examples of hair conditioners include certain cationic polymers, examples of which are:

Polyquaternium-10 (CTFA), available as Polymer JR125 (low molecular weight) and Polymer JR30M (high molecular weight) (both ex Union Carbide) are quaternised hydroxyethyl cellulose.
Guar hydroxypropyl trimonium chloride (CTFA), available as Jaguar-C135.
Polyquaternium-7 (CTFA) a copolymer of acrylamide and dimethyl dialkyl ammonium chloride, available as Merquat 550.
Cocodimonium hydroxy ethyl cellulose, available as Crodacel QM.
Cocodimonium hydrolysed animal protein, available as Crouat M.
Cocodimonium hydrolysed animal keratin, available as Croquat WKP.
Hydrolysed animal keratin, available as Crotein WKP.
Hydrolysed animal protein, available as Crotein SPC.
Hydrolysed silk, available as Crosilk 10,000.
Laurdimonium hydroxy ethyl cellulose, available as Crodacel QL.
Laurdimonium hydrolysed animal protein, available as Croquat L.
Polyquaternium-4 (CTFA) is a copolymer of hydroxyethyl cellulose and diethyl dialkyl ammonium chloride, and is available as Celquat L200.
Steardimonium hydroxy ethyl cellulose, available as Crodacel QS.
Steartrimonium hydrolysed animal protein (CTFA), available as Crotein Q.
Adipic acid/dimethylamino hydroxypropyl diethylenetriamine copolymer, available as Cartaretin.
Polyquaternium-2 (CTFA), available as Mirapol A-15.
Polyquaternium-6 (CTFA) is poly (dimethyl diallyl ammonium chloride) and is available as Merquat 100.
Polyquaternium-11 (CTFA) is a quaternary ammonium polymer formed by the reaction of dimethyl sulphate and a copolymer of vinyl pyrrolidone and dimethyl amino ethyl methacrylate, and is available as Gafquat 734, Gafquat 755 and Quaternium-23.
Polyquaternium-16 is a copolymer of polyvinyl pyrrolidone and methoimidazolinium chloride, and is available as Luviquat FC-370, FC-550, FC-905 and HM-552.

Polyquaternium-17, available as Mirapol AD-1.
Polyquaternium-18, available as Mirapol AZ-1.
Polyquaternium-19 is quaternised polyvinyl alcohol, and is available as Arlatone PQ220 (ex ICI).
Polyquaternium-20 (CTFA) is quaternised alkyl polyvinyl alcohol, and is available as Arlatone PQ225.
Polyquaternium-27 is a block copolymer of Polyquaternium-2 and Polyquaternium-17, and is available as Mirapol 9, 95 and 175.
Poly (vinyl pyrrolidone-co-dimethylamino ethyl methacrylate), available as Copolymer 845, 937 and 958.
Poly(methyl-β-propaniodiallylammonium chloride)
Poly(diallylpiperidinium chloride)
Poly(vinyl pyridinium chloride)
Quaternised poly (dimethylaminoethylmethacrylate).
Quaternium-19
Quaternium-23
Quaternium-40
Quaternium-57
Quaternium-22 (CTFA) is gluconamidopropyl dimethyl 2-hydroxyethyl ammonium chloride.
Quaternium-26 (CTFA) which is mink-amidopropyl dimethyl 2-hydroxyethyl ammonium chloride.
Quaternium-33 (CTFA) which is lanolinamidopropyl dimethylethyl ammonium ethosulfate.
Quaternium-60 (CTFA) which is alkylamidopropyldimethylethyl ammonium ethosulfate, where the alkyl group is a blend of lanolin acid and isostearic acid.
Quaternium-80 (CTFA) which is a diquaternary polydimethyl siloxane, and is available as Abil-Quat 3270 and 3272.

The amount of cationic hair conditioning agent that can optionally be present in the composition according to the invention, is normally up to 5%, preferably from 0.2 to 5% by weight.

Antigrease Agents for Hair

The composition which is employed in the method according to the invention can comprise an antigrease agent as a hair benefit substance.

Examples of antigrease agents include inhibitors of sebum production, such as
vitamin A
13-cis-Retinoic Acid
sulphur
selenium disulphide
methionine
biotin
s-carboxymethyl cysteine
s-benzhydryl cysteine
s-trityl cysteine
4-thiazolidine carboxylic acid
thialysine
N-acetyl homocysteine thiolactone
N,N′-sebacoyl dimethicone
thiolane diol
cyproterone acetate
zinc sulphate
3-benzhydryl-thio-2-aminopropionate of methyl hydrochloride, and
capryloyl hydrolysed animal protein.

Further examples of antigrease agents include those that render hair oleophobic to prevent sebum spreading, such as
Pefluorinated nonanoic acid,
Perfluorinated polyethers (also known as Polyperfluoropropylene oxide), having the structure (30):

$$F(C_3F_6O)_nC_2F_5 \qquad (30)$$

where n is from 4 to 500.
Polyfluoropolymethyl isopropyl ether, available as Fomblin HC, having the structure(31):

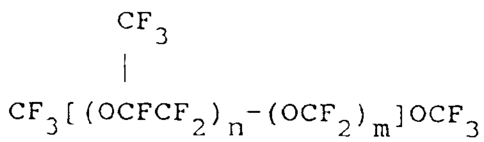 (31)

where n and m each have values of from 20 to 40.

Preferred ethers are those sold under the trade name Fomblin HC by Montefluos. For example, FOMBLIN HC/04 (ave mwt 1500), FOMBLIN HC/25 (ave mol wt 3200), FOMBLIN HC/R (ave mol wt 6600).

Fluorinated polymers obtained from the polymerisation of monomers of the formula (32):

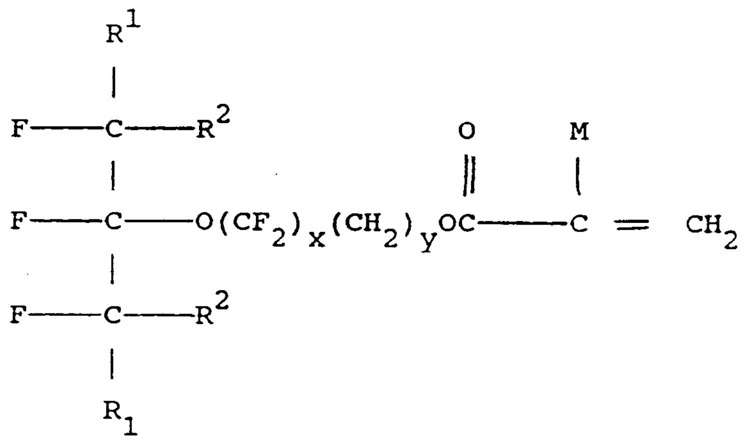 (32)

where $R^1$ and $R^2$ are perhaloalkyl groups having from 1-19 carbon atoms in which all the halogen atoms are from the group consisting of chlorine and fluorine; with the proviso that at least one fluorine is attached to each carbon atom of the $R_1$ and $R_2$ groups, M is H or $CH_3$ and x and y are integers from 1-20 inclusive.

Examples are

$(CF_3)_2CFOCF_2CF_2CH_2CH_2OCOC(CH_3){=}CH_2$ (33)

$(CF_3)_2CFOCF_2CF_2CF_2CF_2CH_2CH_2OCOC(CH_3){=}CH_2$ (34)

$(CF_3)_2CFOCF_2CF_2CF_2CF_2CF_2CF_2CH_2CH_2OCOC(CH_3){=}CH_2$ (35)

An especially preferred monomer of this type is the one having formula 33 above.

The polymerisation reactions are preferably of the emulsion type and the molecular weight of the emulsion polymer can vary, depending on the reaction conditions but a typical molecular weight range is of the order of about 100,000 to about 10,000,000.

Fluorosurfactants having the structure (36):

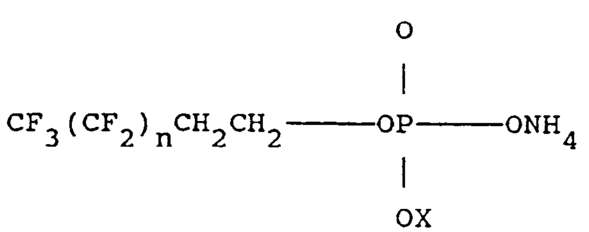 (36)

where X is $NH_4$ or $CH_2CH_2(CF_2)_nCF_3$ n is 0 to 10;

Specific examples of fluorosurfactants include:

Zonyl FSP (ex EI du Pont de Nemours & Co), Zonyl FSA, Zonyl FSN, Lodyne 106B, Lodyne 112B, Fluorads (ex Minnesota Mining and Manufacturing company).

The amount of antigrease agent for hair that can optionally be present in the composition according to the invention, is normally up to 20%, preferably from 1 to 10% by weight.

## Scalp Hygiene Agents

The composition which is employed in the method according to the invention can comprise a scalp hygiene

agent, such as an anti-dandruff agent and bactericide as a hair benefit substance.

Examples of antidandruff agents include:

Selenium disulphide
Zinc pyridinethione
Zinc chelate of S-(N-Oxypyridyl-2)
Magnesium sulphate or calcium chloride complexes of Bis (2-pyridyl-1-oxide) disulphide
L-cysteine
Hexachlorophene
Salicylic acid
Resorcinol
Sodium sulphacetamide
Trichloromethyl-mercapto-4-cyclohexene-2,2-dicarboximide
Tellurium dioxide
Sulphur
Bis-lauryltrimethylammonium polythionate
Octopirox
Zinc pyridinethiol-N-oxide
Undecylenoyl hydrolysed animal protein,
Menthol pantothenate

Examples of bactericides include:

Thymol
Trichlorinated phenol
Trichlorocarbanilide
Chlorosalicylanilide
5,7 Dichloro-8-hydroxyquinoline
Zinc Undecylenate
Polyvinyl pyrrolidone - Iodine complex
2,4,4′-trichloro-2′-hydroxy diphenyl ether, available as Irgasan DP 300.
N-trichloromethyl-thio-3a, 4,7,7a-tetrahydrophthalimide, available as Captan.

The amount of scalp hygiene agent that can optionally be present in the composition according to the invention, is normally present up to 10%, preferably from 0.001 to 5% by weight.

Sunscreen Agents

The composition which is employed in the method according to the invention can comprise a sunscreen agent as a hair benefit substance.

Examples of sunscreen agents which appear in the EEC Cosmetics Directory (Dir. 76/768/EEC - March 1989 Annex VII p.1), and the FDA list of approved sunscreens) include:

| Chemical Name | CTFA Name | Trade Name |
|---|---|---|
| 4-Aminobenzoic acid | PABA | |
| N,N,N-Trimethyl-4-)2-oxoborn-3-ylidenemethyl) anilinium methyl sulphate | | |
| Homomenthyl salicylate | Homosalate | Filtrasol |
| 2-hydroxy-4-methoxybenzophenone | Benzophenone-3 | Uvinul M-40 |
| 3-Imidazol-4-ylacrylic acid and its ethyl ester | | |
| 2-Phenylbenzimidazole-5-sulphonic acid and its potassium, sodium and triethanolamine salts | | |

| Chemical Name | CTFA Name | Trade Name |
|---|---|---|
| N-Propoxylated ethyl-4-aminobenzoate (mixed isomers) | | |
| Ethoxylated ethyl-4-amino-benzoate | | |
| Glycerol 1-(4-amino-benzoate) | Glyceryl PABA | Escalol 106 |
| 2-Ethylhexyl 4-dimethyl-aminobenzoate | Octyl dimethyl PABA | Escalol 507 |
| 2-Ethylhexyl salicylate | Octyl salicylate | Sunarome WMO |
| Isopentyl-4-methoxy-cinnamate (mixed isomers) | | |
| 2-Ethylhexyl 4-methoxy-cinnamate | Octyl methoxy cinnamate | Parsol MCX |
| 2-Hydroxy-4-methoxy-4'-methylbenzophenone | Benzophenone 10 | Contains mexenone (1) |
| 2-Hydroxy-4-methoxybenzo phenone-5 sulphonic acid and sodium salt (Sulisobenzone and Sulisobenzone sodium) | Benzophenone 5 | Spectra sorb UV 284 |

| Chemical Name | CTFA Name | Trade Name |
|---|---|---|
| alpha-(2-Oxoborn-3-ylidene) toluene-4-sulphonic acid and its salts | | |
| 2-ethyloxyethyl p-methoxycinnamate | Cinoxate | |
| 3-(4'-Methylbenzylidene)-d-l-camphor | | Eusolex 6300 |
| 3-Benzylidene camphor | 3-Benzylidene camphor | |
| 4-Isopropyl-dibenzoyl-methane | | |
| 4-Isopropylbenzyl salicylate | Isopropylbenzyl salicylate | Megasol |
| 1-(4-tert-Butylphenyl)-3-(4'-methoxyphenyl) propane-1,3-dione | | |
| 2,4,6-Trianilino-(p-carbo-2'-ethylhexyl-1'-oxi)-1,3,5-triazine | | |
| Diethanolamine-p-methoxy cinnamate | | |
| Digalloyl trioleate | | |

| Chemical Name | CTFA Name | Trade Name |
|---|---|---|
| Ethyl 4-bis (hydroxypropyl) aminobenzoate | | |
| 2-Ethyl-2-cyano-3, 3-diphenylacrylate | | |
| 2-Hydroxy-1,4-naphthoquinone | Lawsone | |
| Methyl anthranilate | | |
| Titanium dioxide | Titanium dioxide | Titanox 2010 |
| Triethanolamine salicylate | TEA-Salicylate | Sunarome W |

The amount of sunscreen agent that can optionally be present in the composition according to the invention, is normally up to 10%, preferably from 0.1 to 5% by weight.

Penetration Enhancers

The composition which is employed in the method according to the invention can comprise penetration enhancer further to potentiate the benefit of the hair benefit substance, by improving its delivery through the stratum corneum to its site of action in the immediate environment of the hair follicle close to the dermal papilla.

The penetration enhancer can accordingly function in a variety of ways. It can for example, improve the distribution of the hair benefit substance on the skin surface or, it can increase its partition into the skin from the composition when applied topically, so aiding its passage to its site of action. Other mechanisms enhancing the benefit of the hair benefit agent may also be involved.

Examples of penetration enhancers include:

2-methyl propan-2-ol
Propan-2-ol
Propane-1,2-diol
dipropylene glycol mono methyl ether
Ethyl-2-hydroxypropanoate
Hexan-2,5-diol
POE(2) ethyl ether
Di(2-hydroxypropyl) ether
Pentan-2,4-diol
Acetone
POE(2) methyl ether
2-hydroxypropionic acid
2-hydroxyoctanoic acid
Propan-1-ol

1,4 Dioxane
Tetrahydrofuran
Butane-1,3-diol
Butane-1,4-diol
Propylene glycol dipelargonate
Polyoxypropylene 15 stearyl ether
Octyl alcohol
POE ester of oleyl alcohol
Oleyl alcohol
Lauryl alcohol
Dioctyl adipate
Dicapryl adipate
Diisopropyl adipate
Diisopropyl sebacate
Dibutyl sebacate
Diethyl sebacate
Dimethyl sebacate
Dioctyl sebacate
Dibutyl suberate
Dioctyl azelate
Debenzyl sebacate
Dibutyl phthalate
Dibutyl azelate
Ethyl myristate
Dimethyl azelate
Butyl myristate
Dibutyl succinate
Didecyl phthalate
Decyl oleate
Ethyl caproate
Ethyl salicylate
Isopropyl palmitate
Ethyl laurate
2-ethyl-hexyl pelargonate
Isopropyl isostearate
Butyl laurate
Benzyl benzoate
Butyl benzoate
Hexyl laurate
Ethyl cabrate
Ethyl caprylate
Butyl stearate
Benzyl salicylate
2-hydroxypropanoic acid
2-hyroxyoctanoic acid,
Decylmethyl sulphoxide
Dimethyl sulphoxide
N,N-Dimethyl acetamide
N,N-Dimethyl formamide
2-Pyrrolidone
1-Methyl-2-pyrrolidone
5-Methyl-2-pyrrolidone
1,5-Dimethyl-2-pyrrolidone
1-Ethyl-2-pyrrolidone
Phosphine oxides
Sugar esters
Tetrahydrofurfural alcohol
Urea

Diethyl-m-toluamide, and
1-Dodecylazacyloheptan-2-one.

Further examples of penetration enhancers include

2-Hydroxyalkanoic acids having from 3 to 28 carbon atoms, preferred examples of which include 2-hydroxpropanoic acid, 2-hydroxyhexanoic acid and 2-hydroxyoctanoic acid; and;

Acid-soap complexes of 2-hydroxyalkanoic acids having from 6 to 28 carbon atoms, preferred examples of which have an elemental analysis of (40):

$$(C_m H_{2m-\frac{1}{2}} O_3) (C_n H_{2n-\frac{1}{2}} O_3) M \qquad (40)$$

where m and n have the same or different values, and each is an integer of from 6 to 28, and M is a cation. The cation M is a monovalent ion such as potassium, sodium or ammonium. A particularly preferred example of the acid-soap complex is that derived from two molecules of 2-hydroxyoctanoic acid which has the empirical formula $C_{16}H_{31}O_6Na$.

The amount of hair benefit substance which can be present in the composition according to the invention is from 0.1 to 50%, preferably from 0.5 to 25% by weight of the composition.

## The cosmetically acceptable vehicle

The method according to the invention accordingly also usually involves the use of a cosmetically acceptable vehicle to act as a diluent, dispersent or carrier for the ester of nicotinic acid so as to facilitate its distribution on the hair and/or the scalp of the user.

Vehicles other than water can include liquid or solid emollients, solvents, humectants, thickeners and powders. Examples of each of these types of vehicle, which can be used singly or as mixtures of one or more vehicles, are as follows:

Emollients, such as stearyl alcohol, glyceryl monoricinoleate, glyceryl monostearate, mink oil, cetyl alcohol, isopropyl isostearate, stearic acid, isobutyl palmitate, isocetyl stearate, oleyl alcohol, isopropyl laurate, hexyl laurate, decyl oleate, octadecan-2-ol, isocetyl alcohol, eicosanyl alcohol, behenyl alcohol, cetyl palmitate, silicone oils such as cyclodimethyl siloxanes dimethylpolysiloxane, di-n-butyl sebacate, isopropyl myristate, isopropyl palmitate, isopropyl stearate, butyl stearate, polyethylene glycol, triethylene glycol, lanolin, cocoa butter, corn oil, cotton seed oil, tallow, lard, olive oil, palm kernel oil, rapeseed oil, safflower seed oil, evening primrose oil, soybean oil, sunflower seed oil, avocado oil, olive oil, sesame seed oil, coconut oil, arachis oil, castor oil, acetylated lanolin alcohols, petroleum jelly, mineral oil, butyl myristate, isostearic acid, palmitatic acid, isopropyl linoleate, lauryl lactate, myristyl lactate, decyl oleate, myristyl myristate;

Propellants, such as trichlorofluoromethane, dichlorodifluoromethane, dichlorotetrafluoroethane, monochlorodifluoromethane, trichlorotrifluoroethane, propane, butane, isobutane, dimethyl ether, carbon dioxide, nitrous oxide;

Solvents, such as ethyl alcohol, methylene chloride, isopropanol, acetone, ethylene glycol monoethyl ether, diethylene glycol monobutyl ether, diethylene glycol monoethyl ether, dimethyl sulphoxide, dimethyl formamide, tetrahydrofuran;

Powders, such as chalk, talc, fullers earth, kaolin, starch, gums, colloidal silica sodium polyacrylate, tetra alkyl and/or trialkyl aryl ammonium smectites, chemically modified magnesium aluminium silicate, organically modified montmorillonite clay, hydrated aluminium silicate, fumed silica, carboxyvinyl polymer, sodium carboxmethyl cellulose, ethylene glycol monostearate.

The cosmetically acceptable vehicle will usually form from 10 to 99.9%, preferably from 50 to 99% by weight of the composition and can, in the absence of other cosmetic adjuncts, form the balance of the composition.

## Surfactants

The composition for use in the method according to the present invention comprises at least 1% by weight of a cosmetically acceptable surfactant, selected from anionic, cationic, nonionic, amphoteric and zwitterionic surfactants or mixtures thereof suitable for application to the body and/or hair. The surfactant may be included in addition to the cosmetically acceptable vehicle or may itself provide the cosmetically acceptable vehicle or be an element of it.

Compositions suitably include not more than 30% by weight of surfactant perferably not more than 20%. A typical shampoo might contain between 5 and 25% by weight of a surfactant, preferably from 7 to 20% by weight. Where the composition is for use as a hair conditioner it might include from 1 to 10% by weight, preferably 1 to 8%, particularly preferably from 3 to 8% by weight of surfactant.

Suitable cationic surfactants have already been exemplified above under the heading "hair conditioners". Examples of other surfactants suitable for inclusion in a composition for application to the body surface, and

in particular to the hair and/or scalp as a hair shampoo are set out below.

Anionic surfactant

The composition of the invention may comprise an anionic surfactant which is preferably chosen from alkyl sulphate, alkyl ether sulphate, alkyl sulphonate, alkyl aryl sulphonate, olefin sulphonate, acyl sarcosinate, acyl tauride, acyl isethionate, nonoalkyl sulphosuccinate, dialkylsulphosuccinate, acryl lactylate, acylated α-amino acid, alkyl carboxylate, monoalkyl phosphate and dialkyl phosphate.

Suitable examples of anionic surfactants which may be included in the compositions of the invention include:

alkyl sulphates, such as sodium lauryl sulphate [eg. EMPICOL CX available from Albright & Wilson], and triethanolaminde lauryl sulphate [eg. EMPICOL TL40/T, available from Albright & Wilson].

alkylether sulphates, such as sodium lauryl ether sulphate [eg. EMPICOL ESB70, available from Albright & Wilson].

alkyl sulphonates, such as sodium alkane ($C_{13-18}$) sulphonate [eg. HOSTAPUR SAS 30, available from Hoechst].

alkylaryl sulphonates, such as sodium alkyl benzene sulphonate [eg. TEEPOL CM44, available from Shell].

olefin sulphonates, such as sodium olefin sulphonate ($C_{5-18}$) [eg. HOSTAPUR OS, available from Hoechst].

acyl sarcosinates, having the structure: (51)

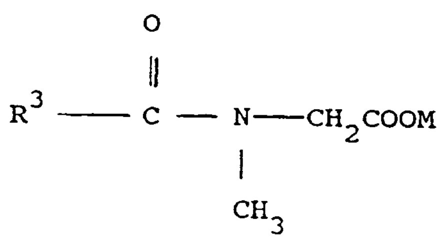

(51)

where $R^3$ is chosen from $C_{6\ 14}$ alkyl, and

M is a counterion chosen from alkali metals, ammonium and substituted ammonium such as alkanolammonium.

An example of an acyl sarcosinate having the structure (51), is sodium lauryl sarcosinate [eg. HAMPOSYL L-95, available from Grace].

acyl taurides, having the structure (52):

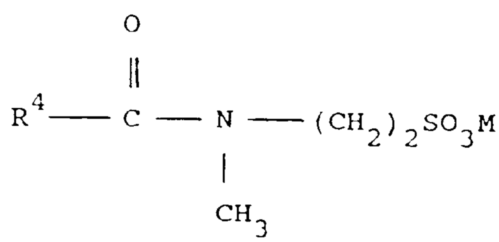

(52)

where $R^4$ is chosen from $C_{8-18}$ alkyl

An example of an acyl tauride having the structure (52) is coconut methyl taurine [eg. FENOPEN TC 42, available from GAF].

acyl isethionates, having the structure (53):

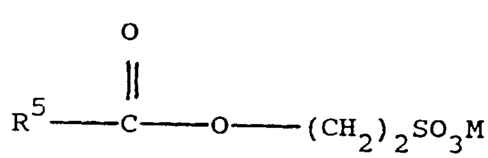

(53)

where $R^5$ is chosen from $C_{8-18}$ alkyl.

An example of an acyl isethionate having the structure (53) is sodium acyl isethionate [eg. JORDAPON C1, available from Jordon].

monoalkyl sulphosuccinates, having the structure (54):

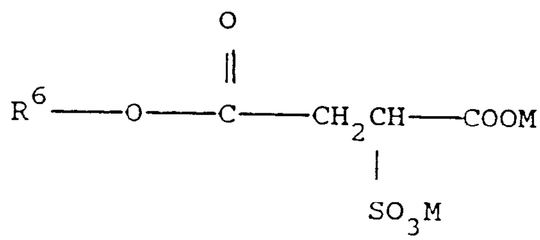

(54)

where $R^6$ is chosen from $C_{10-20}$ alkyl.

Examples of monoalkyl sulphosuccinates having the structure (54) include:

sodium lauryl sulphosuccinate [eg EMPICOL SLL, available from Albright & Wilson)

magnesium alkyl sulphosuccinate (eg. ELFANOL 616 Mg, available from AKZO]

sodium lauryl ethoxysulphosuccinate [eg. EMPICOL SDD, available from Albright & Wilson]

coconut monoethanolamide ethoxysulphosuccinate [eg. EMPICOL SGG]

disodium lauryl polyglycolether sulphosuccinate [eg. SURTAGENE S30, available from CHEM-Y]

polyethyleneglycol sulphosuccinate [eg. REWOPOL SBFA 30, available from REWO].

dialkyl sulphosuccinates, having the structure (55):

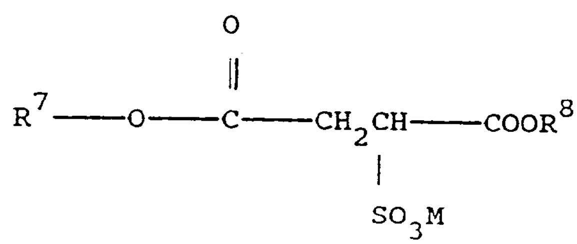

(55)

where $R^7$ and $R^8$ are the same or different, and are chosen from $C_{6-14}$ alkyl.

An example of a dialkyl sulphosuccinaie having the structure (55) is sodium dilauryl sulphosuccinate [eg. EMCOL 4500, available from Witco].

acyl lactylates, having the structure (56):

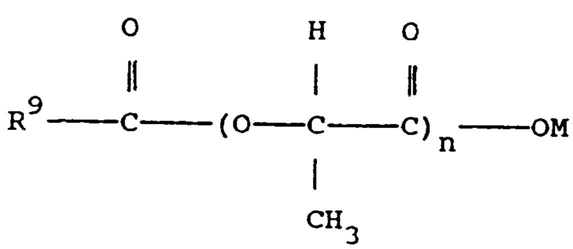

(56)

where $R^9$ is chosen from $C_{6-16}$ alkyl,

and n is 1 or 2.

An example of an acyl lactylate having the structure (6) is decanoyl lactylate [eg. PATIONIC 122A, available from Patterson, CJ].

acylated α-amino acids, such as sodium lauroyl glutamate [eg. ACYLGLUTAMATE LS-11, available from Ajinomoto Co. Inc]

ethyl carboxylates, such as alkyl $C_{12-14}O(EO)_4OCH_2CO_2Na$[eg. AKYPO RLM 38, available from Akzo].

monoalkyl phosphates and dialkyl phosphates, such as dioctyl phosphate.

Amphoteric surfactant

The compositions of the invention may comprise one or more amphoteric surfactants. Suitable amphoteric surfactants are derivatives of aliphatic quaternary ammonium, phosphonium and sulphonium compounds, wherein the aliphatic radicals contain from 8 to 18 carbon atoms, and may be straight chain or branched, and further contain an anionic water-solubilising group, such as carboxyl, sulphonate, sulphate, phosphate or phosphonate.

Preferred amphoteric surfactants include -

Alkyl betaines, having the structure (57):

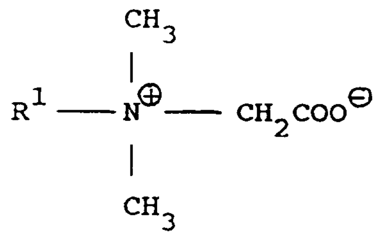

(57)

where $R^1$ is $C_{1-16}$ alkyl.

An example of an alkyl betaine having the structure (7) is lauryldimethyl betaine (eg. EMPIGEN BB, available from Albright & Wilson).

Alkylamidopropyl betaines, having the structure (58):

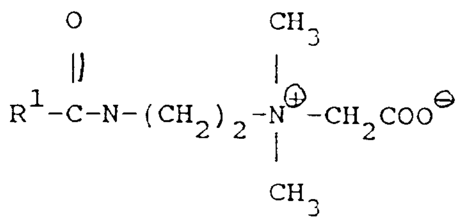

(58)

An example of an alkylamidopropyl betaine having the structure (58) is cocamidopropyl betaine (eg. TEGOBETAIN L7, available from Goldschmidt).

Alkylamphoglycinates or Alkylamphopropionates having the structure (59):

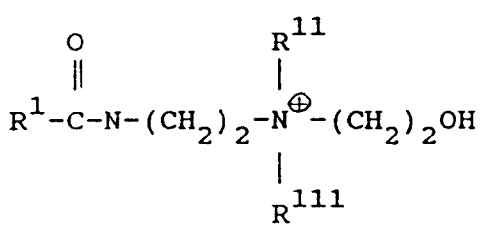

(59)

$R^{11}$ is chosen from H, $CH_2COO^{\ominus}$ and $(CH_2)_2COO^{\ominus}$, and

$R^{111}$ is chosen from $CH_2COO^{\ominus}$ and $(CH_2)_2COO^{\ominus}$

Suitable examples of compounds (59) are cocoamphoglycinate (available from GAF), and cocoamphopropionate.

Sultaines, having the structure (60):

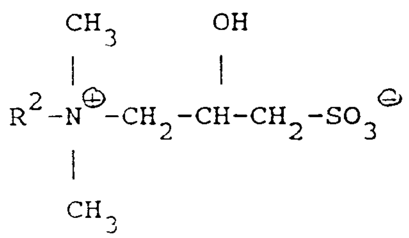 (60)

where $R^2$ is chosen from $C_{12-16}$ alkyl alkylamido.

An example of a sultaine having the structure (60) is cocamidopropylhydroxysultaine (eg. CYCLOTERIC BET-CS, available from Alcolac).

The most preferred amphoteric surfactant are lauryl dimethyl betaine and cocamidopropyl betaine.

Such amphoteric surfactants can contribute to the foaming of the shampoo of the invention, while ameliorating the harshness of the anionic surfactant.

Nonionic surfactant

The composition of the invention may comprise one or more alkoxylated or glycosidic nonionic surfactant having an HLB of 8 or more. Above this value nonionics generally form clear isotropic solutions in combination with the other surfactants in the ranges defined above. Preferred nonionic surfactants are polyoxyethylene alkyl esters and polyoxyethylene alkyl ethers and alkyl polyglycosides.

A suitable example of a polyoxyethylene alkyl ester is that having the CTFA designation Polysorbate 80 which is a mixture of oleate esters of sorbitol and sorbitol anhydrides, condensed with approximately 20 moles of ethylene oxide. Also suitable is Polysorbate 20 which is a mixture of laurate esters of sorbitol and sorbitol anhydrides condensed with approximately 20 moles of ethylene oxide.

Polysorbate 80 and Polysorbate 20 are available commercially as TWEEN 80 and TWEEN 20 respectively, from ICI Americas.

Also suitable for use in the compositions of the invention is the polyethylene glycol ether of $C_{9-11}$ alcohol with an average of 8 ethoxy units, which is available commercially as NONIDET LE-8T or as SYNPERONIC 91-8T, and the polyethylene glycol ether of $C_{12-15}$ alcohol with an average of 9 ethoxy units which is available commercially as DOBANOL 25-9.

Particularly useful alkyl polyglycosides include the glycosides of glucose or glucose oligomers where the alkyl chain can be $C_{8-16}$ and the average number of glucose units is 1 to 2. A suitable example is ORAMIX NS 10 which is the glucoside of $C_{10-12}$ fatty alcohol with an average of about 1.5 glucose units.

Further ingredients

The composition according to the invention can also comprise minor amounts of other ingredients such as anti-bacterial agents, foam boosters, pearlescers, perfumes, dyes, colouring agents, preservatives, thickeners, proteins, polymers, phosphate esters, and buffering agents.

The amount of other ingredients present in the composition according to the invention can form the balance of the composition.

Preservation of the Composition

The composition for use in the method according to the invention is preferably preserved in such a manner that it will enjoy an extended shelf life following manufacture and prior to sale and use. Ideally the composition will have an indefinite shelf life.

It is accordingly apparent that elements of the composition may be prone to attack by bacteria, moulds and fungi and other microbial influences, particularly at pH values near that of the skin that characterise the preferred composition. The shelf-life of the composition can therefore be unacceptably short due to its biodegradation unless steps are taken to preserve the composition.

In order to be preserved, the composition should preferably be free, or substantially free, from viable microbial contaminants that are capable of resulting in microbial spoilage of the composition, and/or its biodegradation prior to topical application of the composition to mammalian skin or hair. It is to be understood, however, that the invention is also concerned with compositions, as herein defined, which may contain viable but dormant

microorganisms, such as bacterial spores, provided that the conditions of preservation do not result in substantial proliferation of the microorganisms prior to use of the composition.

Examples of methods that can be employed to achieve preservation of the composition, includes the following:

(i) Sterilisation

The composition according to the invention can be preserved by sterilisation to remove or kill substantially all viable microbial contaminants. This can be achieved for example by irradiation using a lethal dose of gamma rays, by heat sterilisation or by ultrafiltration using techniques that are well established in the pharmaceutical industry.

(ii) Chemical Preservative

The composition according to the invention can also be preserved by including in it a chemical preservative which functions to prevent the growth of or kill bacteria, fungi or other microorganisms.

Examples of chemical preservatives include ethanol, benzoic acid, sodium benzoate, sorbic acid, potassium sorbate, sodium propionate and the methyl, ethyl, propyl and butyl esters of p-hydroxybenzoic acid. The amount of chemical preservative that can be incorporated in the composition according to the invention will generally be from 0.05 to 5%, preferably from 0.1 to 2% by weight, the amount chosen being sufficient to arrest microbial proliferation.

(iii) Water activity depressants

The composition according to the invention can also be preserved by the inclusion of a water activity depressant such as glycerol, propylene glycol, sorbitol, sugars and salts, for examples alkali metal halides, sulphates and carboxylates. When employing a water activity depressant, sufficient should be incorporated in the composition according to the invention to reduce the water activity ($\alpha_w$) from 1 to $< 0.9$, preferably to $< 0.85$ and most preferably $< 0.8$, the lowest of these values being that at which yeasts, moulds and fungi will not proliferate.

## DEFINITION OF THE INVENTION : (COMPOSITION)

The invention also provides a composition suitable for topical application to the surface of the human body, in particular to the hair and/or the scalp of the head of a human subject which composition is intended subsequently to be rinsed from the body surface with water by a method in accordance with the method claimed as herein described, which composition comprises an amount of an ester of nicotinic acid having the structure (1) sufficient to produce a therapeutic and/or sensory response on the scalp which persists after rinsing.

## DISCLOSURE OF THE INVENTION : (COMPOSITION)

The composition of the present invention comprises a nicotinic acid ester of formula (1) and at least 1% by weight of a surfactant. Optionally the composition further comprises a cosmetically acceptable vehicle. A preferred composition according to the invention is suitable for topical application to the hair and/or the scalp of the head of a human subject and is intended subsequently to be rinsed from the head with water by the method as hereinbefore defined.

Other ingredients including hair benefit substances, particularly hair growth promoters, together with other adjuncts as desired can be present in the composition in accordance with the invention and are generally as hereinbefore disclosed in the description of the method according to the invention. In general such benefit substances will be present in an amount such that a benefit may be achieved even after rinsing.

## Process

The invention also provides a process for the preparation of a composition suitable for topical application to the surface of the body, in particular to the human hair or scalp which comprises mixing a nicotinic acid ester of formula (1) with a surfactant and, optionally a suitable vehicle to provide a composition according to the invention, in which the nicotinic acid ester forms from 0.00001 to 99% by weight of the composition.

Product Form and Container

The compositions of the invention can be formulated as liquids, for example as a lotion, shampoo, milk or cream for use in conjunction with an applicator such as a roll-ball applicator, or a spray device such as an aerosol can containing propellant, or a container fitted with a pump to dispense the liquid product. Alternatively, the compositions of the invention can be solid or semi-solid, for example as a stick, powder, creams or gels, for use in conjunction with a suitable applicator or simply a tube, bottle or lidded jar, or as a liquid-impregnated fabric, such as a tissue wipe.

The invention accordingly also provides a closed container containing a composition as herein defined.

Use of the composition

The invention also provides for the use of the composition as herein defined, for topical application to the body surface, in particular to the hair and/or scalp for inducing a sensory or therapeutic response to the body surface or scalp.

For example, a composition according to the invention can accordingly be used to induce a tingling or warming sensation to the scalp following topical application to the hair or scalp and following rinsing of the composition from the head of the human subject.

The composition of the invention can also be employed to induce a response from the scalp, in particular to induce hair growth, which effect is observed even after rinsing the composition from the head. More specifically, the invention provides for the use of at least 0.00001% by weight of an ester of nicotinic acid having the structure (1) as an agent for producing a sensory response to the body surface eg scalp of the human subject, in a composition comprising a major proportion of a cosmetically acceptable vehicle for the ester, the sensory response persisting after the scalp has been rinsed with water.

The invention also more particularly provides for the use of at least 0.00001g by weight of an ester of nicotinic acid having the structure (1) as an agent for promoting, maintaining or enhancing hair growth in a shampoo comprising a cosmetically acceptable surfactant as a vehicle for the ester of nicotinic acid.

The invention also more particularly provides for the use of such a shampoo which further comprises a second hair growth promoter which together with the ester of nicotinic acid provides an agent for promoting, maintaining or enhancing hair growth.

The compositions according to the invention are primarily intended for topical application to the scalp of the human subject, particularly where the head is already bald or balding, in order to promote the regrowth of terminal hair. The compositions can also be applied prophylactically to the hair and hence the scalp to reduce or prevent the onset of baldness.

The amount of the composition and the frequency of application to the hair and/or scalp can vary widely, depending on personal needs, but it is suggested as an example that topical application of from 0.1 to 5g daily containing from 0.00001 to 1g of a selected chemical inhibitor over the period of at least six months will in most cases result in an improvement in hair growth.

EXAMPLES

The invention is illustrated by the following examples in which shampoo compositions are described, each of which when employed in the method of the invention give rise to some hair benefit as indicated in each case. Following application to the hair and scalp in accordance with standard shampoo procedure in which the head is rinsed with water, a tingling or warming sensation on the scalp is felt by the user and this is to be taken as a signal that a hair benefit (e.g. conditioning, scalp hygiene, hair growth) has been or is being achieved.

Example 1

This example illustrates a shampoo in accordance with the invention which in use is intended to promote or at least maintain hair growth.

| Ingredient | % w/w |
|---|---|
| Sodium lauryl ether sulphate (70% AD) | 35.0 |
| Coconut diethanolamide | 2.0 |
| Guar hydroxypropyl trimonium chloride | 0.2 |
| Citric acid | 0.1 |
| Sodium chloride | 0.8 |
| Preservatives | 0.1 |
| Ethyl nicotinate | 2.0 |
| D-glucaro-1,4-lactone | 5.0 |
| Water | to 100.0 |

Example 2

This example illustrates an anti-dandruff shampoo in accordance with the invention which in use is intended to reduce, eliminate or at least prevent dandruff development on the hair and scalp, while at the same time maintaining hair growth or promoting new hair growth.

| Ingredient | % w/w |
|---|---|
| Sodium lauryl ether sulphate (70% AD) | 20.0 |
| Coconut diethanolamide | 2.0 |
| Citric acid | 0.3 |
| Sodium chloride | 0.5 |
| Antioxidant | 0.1 |
| Octopirox | 0.9 |
| Ethylene glycol distearate | 5.0 |
| Preservatives | 0.1 |
| Benzyl nicotinate | 3.0 |
| Minoxidil | 1.5 |
| Methionine | 2.0 |
| Water | to 100 |

Example 3

This example illustrates a conditioning shampoo in accordance with the invention which in use is intended to leave the hair in a conditioned state, while at the same time maintaining or increasing hair growth.

| Ingredient | % w/w |
|---|---|
| Sodium lauryl ether sulphate (70% AD) | 24.0 |
| Aminoproyl betaine | 8.0 |
| Amino oxide | 4.0 |
| Guar hydroxypropyl trimonium chloride | 0.3 |
| Citric acid | 0.1 |
| Sodium chloride | 0.4 |
| Preservatives | 0.2 |
| Diethanolamine | 0.1 |
| Phosphate ester | 0.2 |
| Iso-propyl nicotinate | 4.0 |
| Disodium glucosaccharic acid | 5.0 |
| PEG-3 distearate | 2.0 |
| Water | to 100 |

Example 4

This example illustrates a mild shampoo in accordance with the invention, which in use is intended to promote or at least maintain hair growth.

| Ingredient | % w/w |
|---|---|
| Magnesium lauryl ether sulphate | 18.0 |
| Lauryl dimethyl betaine | 7.0 |
| Citric acid | 0.1 |
| Sodium chloride | 1.3 |
| Preservatives | 0.2 |
| Inositol mononicotinate | 0.5 |
| α-tocopheryl nicotinate | 2.0 |
| D-glucaro-1,5-lactam | 3.0 |
| Pyroglutamic acid n-octyl ester | 2.0 |
| Water | to 100 |

Examples 5 and 6 illustrate shampoos suitable for overall body use, including on the hair and having invigorating properties.

Example 5

| Ingredient | % |
|---|---|
| Sodium lauryl ether sulphate (70%) | 17 |
| Lauryl dimethyl betaine | 7 |
| Cyclomide | 1 |
| Citric acid | 0.1 |
| Sodium chloride | 1.3 |
| Ethyl nicotinate | 0.1 |
| Colouring material | 0.02 |
| Preservatives | 0.2 |
| Water | to 100 |

Example 6

This composition was as for Example 5 except that 0.1% by weight benzyl nicotinate replaced ethyl nicotinate.

Example 7

RESPONSES ON HUMAN SKIN OF DOSES OF BENZYL NICOTINATE IN SHAMPOO FROM SHORT CONTACT TIMES.

Thirty-four subjects, equal numbers of male and female were used in a study to investigate the effects of short contact times of diluted shampoo solutions. The solutions which were used were as specified in Example 6 except that the concentration of benzyl nicotinate was varied as shown in the tables and the solutions were ten-times diluted with water from the initial concentration indicated in the tables. The solutions were applied to a series of absorbant pads (A1-test, Imeco AB, Sweden) in 40μl aliquots. The pads were left in contact with the neck for either 20 or 60 seconds. Thereafter the pads were removed, excess solution dried on tissue then rinsed with a moist tissue and finally dried with tissue.

Six concentrations were applied on pre-determined sites for 60 seconds and four concentrations at the higher end of the scale were applied for 20 seconds. The sites had been marked using a template and the distribution of the concentrations were arranged in a series of latin square designs so that all sites had an equal number of concentrations.

Prior to the start of the experiment, each site was rinsed with damp tissue and dried. Then measurements were made on those sites by visual assessment on a defined scale, the redness was also measured by means of an erythema meter, which recorded the absorption of green light from a laser beam due to the blood pigments. Further the change in blood flow velocity was estimated with a laser Doppler flowmeter pF2 (Perimed). Because this technique tales a reasonable time to settle, only selected sites were measured. As soon as the sites had been treated, the three types of readings were taken in the described order and then at five-minute intervals for the next half an hour.

The data was processed and averaged over the thirty-four people. Plots of the various responses were made over the time period. Many of the maximum values were significantly greater than the baseline value and are shown in table A. These same values were also used for the calculation of the regression line of the response against the logarithm to the base ten of the concentration.

| CONC | n PEOPLE | VISUAL RED | | ERYTHEMA METER | | BLOOD FLOW |
|---|---|---|---|---|---|---|
| | | 60 | 20 | 60 | 20 | 20 secs |
| 0.014 | 34 | NS | - | NS | - | - |
| 0.028 | 34 | NS | - | NS | - | - |
| 0.07 | 34/11 | 0.001 | NS | 0.05 | NS | NS |
| 0.14 | 34 | 0.001 | 0.001 | 0.001 | 0.001 | 0.001 |
| 0.28 | 34 | 0.001 | 0.001 | 0.001 | 0.001 | 0.001 |
| 0.7 | 34 | - | 0.001 | - | 0.001 | 0.001 |
| 1.4 | 23 | - | 0.001 | - | 0.001 | 0.001 |

Table A. The maximum values were compared with the baseline and the p-values are shown.

The equations of these regression lines were:-

Visible Redness (20 secs) = 2.68 + 2*LOG(conc %) r = 0.96
Visible Redness (60 secs) = 4.37 + 2.74*LOG(conc %) r = 0.98
Erythema (Meter) (20 secs) = 1.09 + 0.76*LOG(conc %) r = 0.97
Erythema (Meter) (60 secs) = 1.69 + 1.09*LOG(conc %) r = 0.99
Laser Doppler (20 secs) = 42.63 + 25.9*LOG(conc %) r = 0.97.

In Table B the concentrations at which the responses start are shown as determined by the intercept of the regression line.

| | CONTACT TIME 60 secs | CONTACT TIME 20 secs |
|---|---|---|
| VISUAL RED | 0.025% | 0.045% |
| ERYTHEMA METER | 0.028% | 0.037% |
| BLOOD FLOW (LASER DOPPLER) | - | 0.022% |

Table B. The comparison of the minimum concentration to start a response.

The above results establish that a response can be determined significantly from such preparations with contact times as short as 20 seconds. They also show that when the contact time is extended the response is greater or the minimum concentration to achieve an effect is lower. Whilst there was a difference in the level of response from person to person, the general shape of the curves were very similar for all subjects.

The comparison between the various types of measurement show that they are probably measuring related phenomena.

Example 8

EXPERIMENTS WITH ETHYL NICOTINATE ESTER IN SHAMPOO

Five subjects washed one side of the head, forehead and neck with a shampoo the same as in Example

5 but containing a varying level of ethyl nicotinate for a period ranging from one to five minutes, whilst the other side was washed with a placebo shampoo of corresponding composition. The changes occurring on the various symmetrical sites around the head were assessed with the erythema meter and a "Cyclops" pyrometer in sequence. The readings were continued for between 45 minutes and one hour.

The data showed that initially the placebo site showed a decrease in temperature and no change in colour, the active sites increased the redness and the temperature either rose or did not fall so low as the placebo sites. The fall in temperature was caused by the cooling due to the evaporation of the last remnants of water. The greatest changes in redness and temperature were observed on the forehead and neck whilst a lower but significant effect was also noted on the crown of the head. In addition to immediate sensations on the forehead and crown,longer term sensations of a pleasant nature could be felt for up to two hours after treatment on the crown of the head.

The extent of the responses were dependent upon the concentrations of the active in the shampoo and the duration of the contact time, before the head was thoroughly rinsed.

Thirty subjects have used these shampoos containing various levels of the ethyl or benzyl ester under conventional usage and have experienced these sensations. Other persons have used the preparations as overall body shampoos (Example 9) and have experienced the characteristic rise in temperature although of varied magnitude on the skin surface and some slight erythema, but other sensory responses were not noted.

Example 9

A subject showered in the conventional way but without shampoo or soap. The redness and temperature of the skin surface was measured at symmetrical points from the mid forearm down the body to the ankle, after the excess water had been removed by light towelling. The person re-showered and between 2 and 3 grammes of shampoo according to Example 5 was streaked down one side of the body in a broad line. The body shampoo was rubbed into a lather periodically during the two minutes. A placebo shampoo of corresponding composition was applied first to one side of the body and then the time started when the shampoo with the ethyl nicotinate was applied to the other side. After two minutes the whole body was showered ensuring that the active preparation did not contaminate the placebo flank.

The redness was measured with the erythema meter whilst the temperature was measured with a Cyclops pyrometer at ten sites on each side. The time was taken as the mean of the two times for each pair of sites. The time course was followed for about 50 minutes. After about 210 minutes the experiment was repeated, but treating the opposite sides. The result shown below are the differences in redness and temperature of the corresponding sites.

The data demonstrated that all the sites showed an increase in redness and temperature. The magnitude and time course varied from site to site, with least activity on the ankle followed by the forearm whilst the biggest changes occurred at the side shoulder, breast and waist. The maximum temperature difference was seven degrees at the waist.

TABLE C

EFFECT OF SHAMPOOING A STREAK OF 0.1% EtNic ALONG THE LENGTH OF THE BODY APPLICATION TO THE LEFT HAND SIDE - ERYTHEMA METER

| TIME | MEAN TIME | FA | UA | BR | SH | WA | HI | TH | CA | AN |
|---|---|---|---|---|---|---|---|---|---|---|
| 0.00 | 0.00 | -0.65 | -0.28 | -0.12 | 0.00 | 0.03 | 0.03 | -0.23 | -0.26 | -0.53 |
| 10.00 | 10.37 | 0.39 | 1.35 | 1.09 | 1.08 | 0.66 | 0.74 | 0.47 | 0.26 | 0.29 |
| 25.00 | 24.98 | 0.00 | 0.67 | 0.71 | 1.06 | 0.44 | 0.31 | 0.43 | 0.41 | 0.56 |
| 38.00 | 38.30 | -0.06 | 0.82 | 0.91 | 0.77 | 0.79 | 0.12 | 0.28 | -0.17 | -0.43 |
| 53.00 | 52.87 | 0.05 | 0.23 | 0.41 | 0.38 | 0.05 | 0.13 | 0.17 | -0.12 | -0.25 |

Note to table

FA = mid forearm

UA = upper arm

BR = side breast

SH = side shoulder

WA = waist

HI = hip

TH = thigh

CA = calf

AN = ankle

## TABLE D

EFFECT OF SHAMPOOING A STREAK OF 0.1% EtNic ALONG THE LENGTH OF THE BODY APPLICATION TO THE LEFT HAND SIDE - TEMPERATURE CHANGE

| MEAN TIME | FA | UA | BR | SH | WA | HI | TH | CA | AN |
|---|---|---|---|---|---|---|---|---|---|
| 0.00 | -0.20 | 0.00 | -1.10 | 0.40 | -0.30 | 0.10 | 0.10 | -0.30 | -0.10 |
| 17.56 | -0.50 | 3.10 | 4.80 | 3.40 | 4.80 | 1.10 | 3.70 | 2.90 | 0.30 |
| 31.56 | 0.20 | 3.60 | 4.80 | 4.50 | 6.80 | 3.70 | 3.20 | 2.30 | -0.20 |
| 44.89 | -0.30 | 2.70 | 4.30 | 3.30 | 6.40 | 3.30 | 2.50 | 1.10 | 0.70 |
| 59.61 | 0.20 | 1.80 | 1.70 | 1.50 | 5.00 | 2.90 | 1.90 | 0.90 | 0.80 |
| 77.21 | 0.00 | 1.40 | 1.00 | 0.30 | 3.60 | 2.20 | 1.30 | 0.50 | 0.30 |

Note to table

FA = mid forearm

UA = upper arm

BR = side breast

SH = side shoulder

WA = waist

HI = hip

TH = thigh

CA = calf

AN = ankle

TABLE E

EFFECT OF SHAMPOOING A STREAK OF 0.1% EtNic ALONG THE LENGTH OF THE BODY APPLICATION TO THE RIGHT HAND SIDE - ERYTHEMA METER

| TIME | MEAN TIME | FA | UA | BR | SH | WA | HI | TH | CA | AN |
|---|---|---|---|---|---|---|---|---|---|---|
| 0.00 | 0.00 | 0.20 | 0.38 | 0.06 | 0.12 | 0.06 | 0.44 | 0.07 | 0.06 | 0.08 |
| 10.00 | 9.79 | -0.28 | 0.89 | 1.20 | 1.81 | 1.16 | 0.92 | 0.87 | 0.87 | 0.62 |
| 26.00 | 26.13 | 0.51 | 1.40 | 1.48 | 1.61 | 1.09 | 0.55 | 0.43 | 0.75 | 0.57 |
| 42.00 | 42.25 | 0.41 | 0.74 | 0.72 | 0.81 | 0.70 | 0.50 | 0.04 | 0.58 | 0.57 |
| 210.00 | 209.78 | 0.65 | 0.28 | 0.12 | 0.00 | -0.03 | -0.03 | 0.23 | 0.26 | 0.53 |

Note to table

FA = mid forearm

UA = upper arm

BR = side breast

SH = side shoulder

WA = waist

HI = hip

TH = thigh

CA = calf

AN = ankle

TABLE F

EFFECT OF SHAMPOOING STREAK OF 0.1% EtNic ALONG THE LENGTH OF THE BODY APPLICATION TO THE RIGHT HAND SIDE - TEMPERATURE CHANGE

| MEAN TIME | FA | US | BR | SH | WA | HI | TH | CA | AN |
|---|---|---|---|---|---|---|---|---|---|
| 0.00 | -0.35 | -0.55 | 0.60 | -0.50 | 1.25 | -0.55 | -0.25 | -0.05 | -0.05 |
| 17.84 | 0.60 | 1.40 | 4.10 | 3.20 | 6.70 | 4.10 | 3.40 | 2.70 | -0.40 |
| 33.68 | 1.10 | 2.10 | 5.60 | 4.20 | 7.30 | 4.30 | 1.90 | 1.90 | 0.00 |
| 49.79 | 0.90 | 1.20 | 4.70 | 2.70 | 6.10 | 2.80 | 0.80 | 1.30 | 0.20 |
| 217.12 | 0.20 | 0.00 | 1.10 | -0.40 | 0.30 | -0.10 | -0.10 | 0.30 | 0.10 |

Note to table

FA = mid forearm

UA = upper arm

BR = side breast

SH = side shoulder

WA = waist

HI = hip

TH = thigh

CA = calf

AN = ankle

## Claims

1. A composition for topical application to the surface of the human body and intended to be rinsed from the surface, the composition comprising:
   (i) an ester of nicotinic acid having the strucuture (1) in an amount sufficient to produce a therapeutic and and/or sensory response at the surface which persists after rinsing

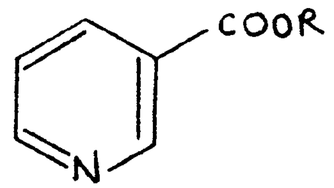

(1)

   branched or unbranched alkyl or alkenyl groups having from 1 to 10 carbon atoms,
   haloalkyl groups having from 1 to 6 carbon atoms,
   alkoxyalkyl groups having from 2 to 6 carbon atoms,
   hydroxyalkyl groups having from 1 to 6 carbon atoms,
   carbamoylalkyl groups having from 2 to 7 carbon atoms,
   alkylaminoalkyl groups having from 2 to 8 carbon atoms,
   aminoalkyl groups having from 1 to 6 carbon atoms,
   cyclohexyl,
   alkylcyclohexyl groups, each alkyl group having from 1 to 4 carbon atoms,
   aryl groups,
   hydroxyaryl groups,
   alcoxyhydroxyaryl groups
   haloaryl groups,
   nitroaryl groups,
   tocopheryl groups,
   inositol; and
   (ii) at least 1% by weight of a cosmetically acceptable surfactant.

2. A composition according to claim 1 containing from 1 to 30% by weight of surfactant.

3. A composltion according to claim 1 or claim 2 in which the ester forms from 0.001 to 20% by weight of the compoistion.

4. A composition according to any one of the preceding claims wherein the ester is selected from ethyl nicotinate, ethyl nicotinate and benzyl nicotinate.

5. A composition according to any one of the preceding claims wherein the surfactant is selected from anionic surfactants, nonionic surfactants, cationic surfactants, zwitterionic surfactants and mixtures thereof.

6. A composition according to any one the preceding claims which is a shampoo.

7. A composition according to any one of the preceding claims for topical application to the hair and/or the scalp and further comprising
   (iii) a hair benefit agent selected from hair growth promoters, hair conditioners, anti-grease agents for hair, scalp hygiene agents, sunscreen agents, styling aids, bodying agents and mixtures thereof.

8. A composition according to claim 7 for producing a therapeutic and/or sensory response on the scalp.

9. A composition according to claim 8 for producing vasodilation at the scalp surface.

10. A composition according to claim 8 or claim 9 for producing an increase in hair growth.

**11.** A composition according to any one of claims 7 to 10 in which the hair benefit agent is a hair growth promoter.

**12.** A composition according to claim 11 in which the hair growth promoter is selected from:

i. D-glucaro-1, 4-lactone,

ii. glucosaccharic acid, disodium salt,

iii. Minoxidil,

iv. $\alpha$-1,4-esterified disaccharide having the structure (2):

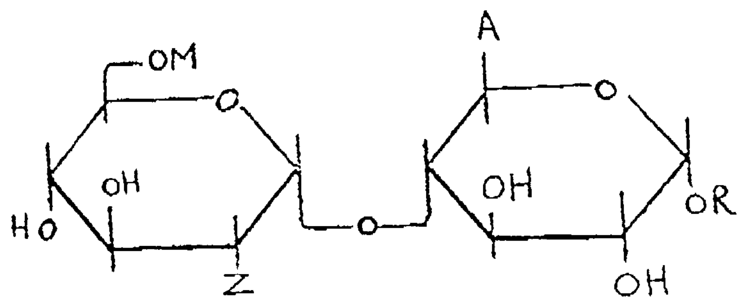

(2)

where

Z represents a functional nitrogen group, such as an azide or a group having the structure -NHB, in which B represents -H or a functional group such as acetyl or sulphate as a salt with an organic or mineral cation;

M represents -H or $SO_3M_1$, where $M_1$ is an organic or metallic cation, particularly an alkali metal; or an acetyl group;

R represents a $C_1$ or $C_4$ alkyl radical, especially methyl; or an aryl radical;

A represents a functional group such as an acid or $-COOR_1$, where $R_1$ represents -H or a $C_1$ or $C_4$ alkyl radical, especially methyl; or a metal, especially an alkali metal;

an oligosaccharide including at least one esterified disaccharide unit consisting of a uronic acid residue having the structure (3):

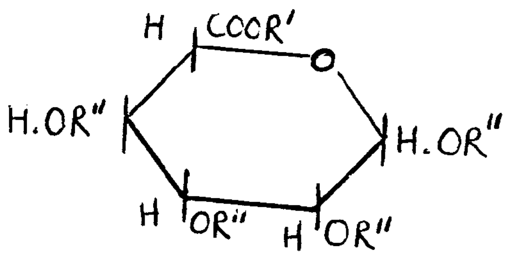

(3)

and a hexosamine residue having the structure (4):

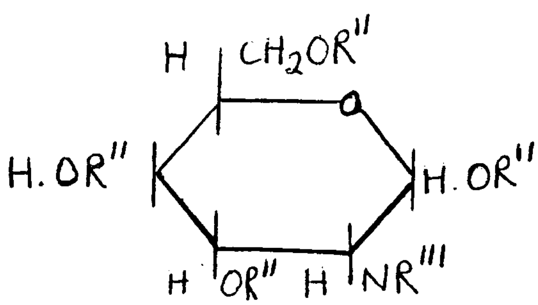

(4)

where

R' is -H, $C_3$ to $C_{10}$ alkyl or

$$\begin{array}{l} \quad COOR'' \\ \quad | \\ -CH(CH_2)_nCH_3; \end{array}$$

R″ is -H, $C_1$ to $C_4$ alkyl,-$CO(CH_2)_mCH_3$,-$SO_3M$; R‴ is -H, -$CO(CH_2)_mCH_3$, or -$SO_3M$,
M is -H, or a metallic or organic cation
n is 0 or an integer of from 1 to 7, and m is 0 or the integer 1 or 2;
the groups designated R″ being the same or different, one R″ group from each pyranose ring structure being linked by a glycosidic linkage having the configuration α-1,3, α-1,4 β-1,4; and the -COOR′, -$CH_2OR''$,
and -OR″ groups being of either configuration with respect to the pyranose rings;
vi. minoxidil glucuronides,
vii. minoxidil sulphates,
viii. N-acetyl glycine
ix. D-glucaro-1,5-lactam,
x. 1,2-dioleoyl-sn-glycerol
xi. 2-propionamido-2-deoxyglucose
xii. pyroglutamic acid n-octyl ester,
xiii. 6-acetyl-galactono-1,4-lactone,
ix. methionine,
x. zinc gluconate,
xi. pentadecanoic acid triglyceride
and mixtures thereof.

**13.** A method of cosmetic treatment of the human body, which comprises the steps of
(i) applying to the body surface a composition according to any one of the preceding claims;
(ii) subsequently rinsing the body surface with water to produce a sensory response which persists after rinsing

**14.** A method according to claim 13 in which the composition is applied to the hair and/or the scalp.

**15.** A method according to claim 13 or 14 wherein the sensory response is a tingling sensation.

**16.** A method according to claim 13 or 14 wherein the sensory response is a warming sensation.

**17.** The use of at least 0.00001% by weight of an ester of nicotinic acid having the structure (1) as an agent for promoting, maintaining or enhancing hair growth, in a shampoo comprising a cosmetically acceptable surfactant as a vehicle for the ester of nicotinic acid.

**18.** The use of at least 0.00001% by weight of an ester of nicotinic acid having the structure (1), together with at least 0.0001 by weight of a hair growth promoter which together provide an agent for promoting, maintaining or enhancing hair growth, in a shampoo comprising a cosmetically acceptable surfactant as a vehicle for the ester and hair growth promoter.

**19.** The use of at least 0.00001% by weight of an ester of nicotinic acid having the structure (1) as an agent for producing a sensory response in the body surface of a human subject, in a composition comprising a major proportion of a cosmetically acceptable vehicle for the ester, the sensory response persisting after the body surface has been rinsed with water.

**20.** A use according to claim 19 in which the agent is for producing a sensory response in the scalp.